Europäisches Patentamt

European Patent Office

Office européen des brevets

(11) Publication number: **0 287 007 B1**

## (12) EUROPEAN PATENT SPECIFICATION

(45) Date of publication of patent specification: **22.01.92**   (51) Int. Cl.⁵: **C07C 67/24**, C07C 69/63

(21) Application number: **88105708.7**

(22) Date of filing: **11.04.88**

(54) Process for cleaving aliphatic ether compounds.

(30) Priority: **14.04.87 DE 3712610**

(43) Date of publication of application:
**19.10.88 Bulletin 88/42**

(45) Publication of the grant of the patent:
**22.01.92 Bulletin 92/04**

(84) Designated Contracting States:
**AT BE CH DE ES FR GB IT LI NL SE**

(56) References cited:
**US-A- 2 190 907**
**US-A- 3 455 996**
**US-A- 4 298 758**

**BULLETIN DE LA SOCIETE CHIMIOUE DE FRANCE, 2nd part, January-February 1974, Paris, FR; J. G. DUBOUDIN et al, "Scission d'éthers-oxydes par le chlorure d'acétyle en présence d'acides de Lewis"**

(73) Proprietor: **THE DOW CHEMICAL COMPANY**
**2030 Dow Center Abbott Road P.O. Box 1967 Midland Michigan 48640-1967(US)**

(72) Inventor: **Eiffler, Jürgen**
**Wagnerstrasse 56**
**W-2160 Stade(DE)**

(74) Representative: **Sternagel, Hans-Günther, Dr. et al**
**Patentanwälte Dr. Michael Hann Dr. H.-G. Sternagel Sander Aue 30**
**W-5060 Bergisch Gladbach 2(DE)**

## Description

Aliphatic ethers which have at least one halogen atom in beta-position to the oxygen atom are obtained as by-products in various industrial processes on a large scale. For example, 1,1'-oxybis-(1-methyl-2-chloroethane) and 1,1'-oxybis(1-chloromethyl-2-chloroethane), commonly known as dichloroisopropyl ether and bis(1,3-dichloroisopropyl) ether are by-products in the chlorohydrin process for making propylene oxide. As evidenced by Ullmann's Enzyklopädie der Technischen Chemie, 4th edition, vol. 19, pages 474 to 480, the worldwide capacity of the propylene oxide plants in which the chlorohydrin process was used was 1,8 million tons in 1980. About 1 to 2 percent dichloroisopropyl ether are produced as a by-product in this reaction, that is, about 18,000 to 36,000 tons have been produced in 1980. However, these by-products do not have a high commercial value and are normally burnt to recover chlorine and their heat values. Therefore, it would be desirable to find a process by which these by-products and other aliphatic ethers having at least one halogen atom in beta-position to the oxygen atom can be converted into useful products.

## Summary of the Invention

It has now been found that these halogenated ethers can be cleaved by reacting the ether with an acid halide in the presence of a Lewis acid catalyst. The corresponding ester and halide which have the unreacted beta-halogen atom(s) are thereby produced.

Accordingly, the present invention relates to a process for cleaving an aliphatic ether compound by contacting it with an acid halide in the presence of a Lewis acid catalyst which process is characterised in that an aliphatic ether having at least one halogen atom in a beta-position to the oxygen atom is cleaved and the corresponding ester and halide having the unreacted beta-halogen atom(s) are produced.

Processes for cleaving an aliphatic ether compound by reacting it with an acid halide in the presence of a Lewis acid catalyst are generally well known, for example from Houben-Weyl, vol. 6/3, page 156, 4th edition, by H. Meerwein and from the Bulletin de la Société Chimique de France, 1974, no. 1-2, pages 272 to 278, by J.-G. Duboudin and J. Valade. It is also known that ethers can be cleaved with an acid anhydride, for example acetic anhydride, in the presence of a Lewis acid catalyst such as FeCl₃; Jerry March, "Advanced Organic Chemistry", 2nd edition, chapter 10, pages 368 to 369. However, the ethers which are cleaved with an acid halide or an acid anhydride described in the literature are not halogenated. In the mentioned literature Bull.Soc.Chim.Fr., 1974, more than 20 examples of aliphatic or cyclic ethers are mentioned which are cleaved with acetyl chloride in the presence of a Lewis acid catalyst, however, not one of these ethers is halogenated. Furthermore, it is a well known text book reaction to produce carbonium ions by reacting halogenated alkanes with Lewis acids such as aluminium chloride. The carbonium ion is highly reactive. For example, it may lose a proton whereby a double bond is created, it can undergo a rearrangement wherein an alkyl or aryl group or a hydrogen migrates to the positive centre or the carbonium ion may add to a double bond in an alkylation reaction (see for example Jerry March, "Advanced Organic Chemistry", 2nd edition, pages 485 to 493 and McGraw-Hill International "Reactions, Mechanisms and Structures", 2nd edition, pages 152 to 160).

Accordingly, one would expect that aliphatic halogenated ethers will undergo a great number of different reactions when they are contacted with a Lewis acid catalyst such as aluminium chloride or FeCl₃. Surprisingly, it has been found that aliphatic ethers which have at least one halogen atom in a beta-position to the oxygen atom, e.g. dichloroisopropyl ether or bis(1,3-dichloroisopropyl) ether, can be cleaved with an acid halide in the presence of a Lewis acid catalyst and the corresponding ester and halide having the unreacted beta-halogen atom(s) are produced at a high selectivity. This is particularly surprising because a high selectivity is not obtained when an acid anhydride is used instead of the corresponding acid chloride. Furthermore, it is known from U.S. patent 4,298,758 to react dichloroisopropyl ether with a carboxylic acid and an alkali metal salt of the carboxylic acid. When using the carboxylic acid instead of the corresponding acid chloride, reaction products which still have unreacted beta-halogen atom(s) are not obtained. According to the process in U.S. patent 4,298,758 propylene glycol diesters and dipropylene glycol diesters are obtained.

## Detailed description of the Invention

According to the present invention aliphatic ethers which have at least one halogen atom in a beta-position to the oxygen atom are cleaved. The halogen atom is preferably chlorine. Preferably, the aliphatic ethers are saturated. Those ethers are preferred which have from 3 to 12, more preferably from 4 to 10 and most preferably from 6 to 8 carbon atoms. The ethers are preferably symmetric, that is, both groups linked to the oxygen atom have the same meaning. Preferred examples are 1,1'-oxybis(2-halogenoC²⁻⁶-alkanes) or 1,1'-oxybis-

(1-halogenomethyl-2-halogenoC$^{2-5}$-alkanes). There-of, 1,1'-oxybis-(l-halogenomethyl-2-halogeno-C$_{2-3}$-alkanes) or 1,1'-oxybis(2-halogeno-C$_{3-4}$-alkanes) are preferred. The most preferred examples thereof are 1,1'-oxybis(l-methyl-2-chloroethane), i.e. dichloroisopropyl ether, and 1,1'-oxybis(1-chloromethyl-2-chloroethane), i.e. bis(1,3-dichloroisopropyl)ether, which are by-products of the chlorohydrin process as described above.

The products obtained in the process of the present invention depend of course on the starting material. Corresponding esters and halides which have the unreacted beta-halogen atom(s) are obtained at high selectivity. For example, when dichloroisopropyl ether is cleaved according to the process of the present invention, the corresponding beta-chloroisopropyl monoester and 1,2-dichloropropane are obtained. 1,1'-oxybis(1-methyl-2-chloroethane) (i.e. dichloroisopropyl ether) which is obtained in the chlorohydrin process is often mixed with minor amounts of isomers of the type 1,1'-oxybis(2-chloropropane) or (2-chloropropyl)(1-methyl-2-chloroethyl)ether. Also these isomers are cleaved to the corresponding esters and halides in the process of the present invention.

The produced monoesters and halides are useful for many applications, for example as starting materials for other processes or as solvents.

Any aliphatic, cycloaliphatic or aromatic acid halide is useful as a reagent in the process of the present invention. Acid chlorides and bromides are preferred. Preferred examples are carboxylic acid chlorides such as cyclohexenyl chloride, benzoyl chloride, ortho-, para- or meta-toluyl chloride or cinnamoyl chloride or sulfonyl chlorides such as ethane sulfonyl chloride, ortho-, para-or meta-toluene sulfonyl chloride. Of the carboxylic acid chlorides and bromides, acetyl chloride, acetyl bromide and pivalic chloride or bromide are preferred. The acid halides may be prepared in situ, for example, acetyl chloride or acetyl bromide can be prepared by the reaction of acetanhydride with anhydrous hydrogen bromide or chloride. The reaction mixture containing additionally acetic acid can be used for the cleaving process of the present invention.

A wide range of Lewis acids are useful as catalyst such as AlBr$_3$, AlCl$_3$, GaCl$_3$ FeCl$_3$ SbCl$_5$ ZrCl$_4$ BCl$_3$ BF$_3$, SbCl$_3$, SnCl$_4$, ZnCl$_2$ or CuCl$_2$ - (preferably anhydrous). The useful amount of the Lewis acid catalyst depends on the various factors such as the starting material for the present invention and mainly on the reactivity of the catalyst. In general, from 0.01 to 2 mols, preferably from 0.1 to 1 mol catalyst, per mol of the aliphatic ether having at least one halogen atom in a beta-position to the oxygen atom, is useful when Lewis acids such as ZnCl$_2$ or FeCl$_3$ are used.

In general, the reaction is carried out at a temperature between 20°C and 250°C, preferably between 60°C and 200°C, more preferably between 100°C and 200°C. The appropriate reaction temperature depends on various factors such as concentration and reactivity of the catalyst. It is preferred to carry out the process at a low catalyst concentration, e.g. 0.1 mol per mol aliphatic ether at a temperature of from 100°C to 250°C. When the reaction temperature is from 20°C to 60°C, higher catalyst concentrations are generally required, e.g. 1 mol per mol aliphatic ether.

The pressure is not critical. In general, 1 to 25 bar, preferably 5 to 15 bar and more preferably 8 to 12 bar are appropriate.

For carrying out the process of the present invention the Lewis acid catalyst can be added to the acid halide and left at room temperature, preferably with stirring, for an appropriate time period; for example for 30 minutes to 2 hours. The aliphatic ether is then added and the mixture is heated from room temperature to the desired, above-mentioned temperature. The reaction can also be carried out in one step by heating a mixture of the acid halide, the Lewis acid catalyst and the aliphatic ether to the desired temperature. Although the aliphatic ether or the acid halide can be used in excess, it is preferred to use equimolar amounts of the aliphatic ether and the acid halide.

The process of the present invention can be carried out in the presence or absence of an inert solvent. Useful inert solvents are for example aliphatic hydrocarbons such as pentane, hexane or cyclohexane, or inert carboxylic acids such as acetic acid.

It is to be understood that the conversion of the halogenated aliphatic ether according to the present invention depends on various factors such as reaction temperature, amount and reactivity of the catalyst and reaction time. The preferred reaction conditions can be found by those skilled in the art. However, it is very surprising that the halogenated ethers are converted at high selectivity to the corresponding monoesters and halides which still retain the unreacted beta-halogen atom(s). Expected side reactions such as a reaction between the halogen atom(s) in a beta-position to the oxygen atom and the Lewis acid catalyst do not take place to a substantial extent. In general, the desired products are obtained from the halogenated aliphatic ether at a selectivity of more than 80 mol percent, and under appropriate reaction conditions, often even more than 85 mol percent.

The products obtained according to the present invention can be separated from each other in a known manner, for example by distillation. The separated, and possibly purified, products can be used for various purposes, for example as starting material for further reactions. However, it is also possible to subject the product mixture obtained according to the process of the present invention directly to further reactions without isolating the obtained products.

One preferred such reaction is a process for producing a diester of an aliphatic vicinal glycol which process is characterised in that in a first step an aliphatic ether having at least one halogen atom in a beta-position to the oxygen atom is cleaved according to the process of the present invention described above and the reaction products are reacted in a second step with an alkali metal salt or an alkaline earth metal salt of a carboxylic acid or sulfonic acid to the corresponding ester(s). Accordingly, another aspect of the present invention is such a process.

Preferably, the products obtained in the first step are not isolated and purified before a diester is produced in the second step. The alkali metal salt or alkaline earth metal salt used in the second step can be derived from any aliphatic, cycloaliphatic or aromatic carboxylic acid or sulfonic acid. Suitable carboxylic acids have from 2 to 6 carbon atoms, preferably 2 or 3 carbon atoms. Most preferably, the sodium or potassium salt of acetic acid or propionic acid is used. Suitable sulfonic acids are hexane sulfonic acid, dodecane sulfonic acid, hexadecane sulfonic acid or o-, m- or p-toluene sulfonic acid. When in the first step an ether of formula R-O-R is cleaved wherein both radicals R have the same meaning and have a halogen atom in a beta-position to the oxygen atom it is preferred that the acid halide in the first step and the alkali metal salt or alkaline earth metal salt in the second step are derived from the same acid. Thereby a uniform product is obtainable instead of a product mixture. For example, propylene glycol diacetate is produced when cleaving 1,l'-oxybis(l-methyl-2-chloroethane) with acetyl chloride in the first step and reacting the products obtained in the first step with an alkali metal salt or an alkaline earth metal salt of acetic acid in the second step.

In the second step generally an organic liquid reaction diluent is used, for example a glycol, glycol ether or a glycol ester. The most preferred diluents are carboxylic acids, in acid form, which correspond to the carboxylic acid salt employed in the second step. For example, if sodium acetate is employed for producing the aliphatic diesters, acetic acid is most preferably employed as the organic liquid reaction diluent. In general, the second step is carried out at a temperature of from 160°C to 300°C to produce the diester at a reasonable rate of reaction (e.g. 90 percent or more conversion in 12 hours or less) coupled with a high selectivity (e.g. selectivity of more than 80 percent). More preferably, the second step is conducted at a temperature of from 170°C to 280°C and most preferably from 180°C to 260°C. In general, the reaction times in the second step will vary from 0.1 to 12 hours, preferably from 0.5 to 8 hours.

The following examples illustrate the invention and should not be construed to limit its scope. All parts are by weight unless otherwise mentioned.

Example 1 .

64.8 parts dry, powdered $FeCl_3$ are added to 445 parts acetyl chloride. The reaction mixture is stirred under nitrogen in a closed reactor at ambient temperature. After one hour 685 parts 1,l'-oxybis(l-methyl-2-chloroethane) (i.e. dichloroisopropyl ether) are added and the reaction mixture is heated to 170°C. The reaction temperature is maintained for 5 hours at 8 bar pressure. After cooling the reactor to ambient temperature, the reaction products are analysed by gas chromatography. 76 mol percent 1,2-dichloropropane and chloro-acetoxy-propane are obtained, based on the amount of dichloroisopropyl ether. The conversion of dichloroisopropyl ether is 86 mol percent. Accordingly, the selectivity of the reaction to the desired products is 88 mol percent.

Example 2

196 parts acetyl chloride and 340 parts dichloroisopropyl ether are stirred under nitrogen in a teflon-lined autoclave. 27.2 parts zinc chloride ($ZnCl_2$) are added, the autoclave is closed and heated to 170°C. After 5 hours the autoclave is allowed to cool to ambient temperature. The yield of 1,2-dichloropropane and chloro-acetoxy-propane is 59 mol percent, based on the amount of dichloroisopropyl ether, determined according to gas chromatography. 68 mol percent of dichloroisopropyl ether are converted, i.e. the selectivity of the reaction to the desired products is 87 mol percent.

Example 3

196 parts acetyl chloride, 340 parts dichloroisopropyl ether and 272 parts zinc chloride ($ZnCl_2$) are stirred in a glas vessel and heated to 60°C over a period of 5 hours. The reaction mixture is cooled to ambient temperature. The yield of 1,2-dichloropropane and chloro-acetoxy-propane is 72 mol percent, based on the amount of dichloroisopropyl ether. The conversion of dichloroisopropyl ether is 83 mol percent, i.e. the selectivity of the reaction to the desired products is 87 mol percent.

Example 4

61 parts dry, powdered FeCl$_3$ are added to 415 parts acetyl chloride. The reaction mixture is stirred in a closed reactor for 45 minutes. 690 parts dichloroisopropyl ether are added and 1 bar excess pressure is provided with nitrogen. The mixture is heated to 150°C in one hour. The temperature is kept for 2.5 hours at 140°C to 145°C (8.5 bar pressure). After the reaction mixture has cooled to room temperature, 3500 parts acetic acid and 1480 parts sodium acetate are added. The mixture is maintained for one hour at 100°C and for further 3.5 hours at 220°C (8 bar pressure). After the mixture has cooled to room temperature, the solution is filtered and analysed by GC/MS (gas chromatography/mass spectroscopy). The overall yield of propylene glycol diacetate is 795 parts, that means that 62 percent of the dichloroisopropyl ether molecules are converted each into two molecules of propylene glycol diacetate.

## Claims

1. A process for cleaving an aliphatic ether compound by contacting it with an acid halide in the presence of a Lewis acid catalyst, characterised in that an aliphatic ether having at least one halogen atom in a beta-position to the oxygen atom is cleaved and the corresponding ester and halide having the unreacted beta-halogen atom(s) are produced.

2. The process of claim 1, characterised in that the aliphatic ether has from 3 to 12 carbon atoms.

3. The process of claim 1, characterised in that a 1,1'-oxybis(2-halogeno-C$_{2-6}$-alkane) is cleaved.

4. The process of claim 1, characterised in that a 1,1'-oxybis(1-halogenomethyl-2-halogeno-C$_{2-5}$-alkane) is cleaved.

5. The process of claim 1, characterised in that a 1,1'-oxybis(2-halogeno-C$_{3-4}$-alkane) or 1,1'-oxybis(1-halogenomethyl-2-halogeno-C$_{2-3}$-alkane) is cleaved.

6. The process of claim 1, characterised in that 1,1'-oxybis(1-methyl-2-chloroethane) or 1,1'-oxybis(1-chloromethyl-2-chloroethane) is cleaved.

7. The process of any of claims 1 to 6, characterised in that the halogen atom in the ether compound is chlorine and the acid halide is an acid chloride or acid bromide.

8. The process of any of claims 1 to 7, characterised in that acetyl chloride or acetyl bromide is used as an acid halide.

9. A process for producing a diester of an aliphatic vicinal glycol, characterised in that in a first step an aliphatic ether having at least one halogen atom in a beta-position to the oxygen atom is cleaved with an acid halide according to the process of any of claims 1 to 8 and the reaction products are reacted in a second step with an alkali metal salt or an alkaline earth metal salt of a carboxylic acid or sulfonic acid to the corresponding ester(s).

10. The process of claim 9, characterised in that the aliphatic ether is an ether of the formula R-O-R wherein both radicals R have the same meaning and have a halogen atom in a beta-position to the oxygen atom and further characterised in that the acid halide in the first step and the alkali metal salt or alkaline earth metal salt in the second step are derived from the same acid.

## Revendications

1. Procédé pour scinder un éther aliphatique, en le mettant en contact avec un halogénure d'acide, en présence d'un catalyseur acide de Lewis, caractérisé en ce que l'on scinde un éther aliphatique comportant au moins un atome d'halogène en position $\beta$ par rapport à l'atome d'oxygène, et en ce que sont produits l'ester et l'halogénure correspondants, qui comportent le ou les atomes d'halogène en $\beta$ n'ayant pas réagi.

2. Procédé de la revendication 1, caractérisé en ce que l'éther aliphatique comporte de 3 à 12 atomes de carbone.

3. Procédé de la revendication 1, caractérisé en ce que l'on scinde un 1,1'-oxy-bis (2-halogéno-alcane en C$_{2-6}$).

4. Procédé de la revendication 1, caractérisé en ce que l'on scinde un 1,1'-oxy-bis (1-halogénométhyl-2-halogéno-alcane en C$_{2-5}$).

5. Procédé de la revendication 1, caractérisé en ce que l'on scinde un 1,1'-oxy-bis (2-halogéno-alcane en C$_{3-4}$) ou un 1,1'-oxy-bis(1-halogénométhyl-2-halogéno-alcane en C$_{2-3}$).

6. Procédé de la revendication 1, caractérisé en ce que l'on scinde le 1,1'-oxy-bis(1-méthyl-2-chloroéthane) ou le 1,1'-oxy-bis(1-chlorométhyl-2-chloroéthane).

7. Procédé de l'une quelconque des revendications 1 à 6, caractérisé en ce que l'atome d'halogène présent dans l'éther est un atome de chlore et en ce que l'halogénure d'acide est un chlorure d'acide ou un bromure d'acide.

8. Procédé de l'une quelconque des revendications 1 à 7, caractérisé en ce que l'on utilise, comme halogénure d'acide, du chlorure d'acétyle ou du bromure d'acétyle.

9. Procédé de production d'un diester d'un glycol vicinal aliphatique, caractérisé en ce que, dans une première étape, on scinde un éther aliphatique comportant au moins un atome d'halogène en position $\beta$ par rapport à l'atome d'oxygène, à l'aide d'un halogénure d'acide, selon le procédé de l'une quelconque des revendications 1 à 8, et dans une seconde étape, on fait réagir les produits de réaction avec un sel de métal alcalin ou de métal alcalino-terreux d'un acide carboxylique ou d'un acide sulfonique, pour obtenir le ou les ester (s) correspondant (s).

10. Procédé de la revendication 9, caractérisé en ce que l'éther aliphatique est un éther de formule R-O-R dans laquelle les deux groupes R ont la même signification et comportent un atome d'halogène en position $\beta$ par rapport à l'atome d'oxygène, et caractérisé en outre en ce que l'halogénure d'acide utilisé dans la première étape et le sel de métal alcalin ou de métal alcalino-terreux utilisé dans la seconde étape dérivent du même acide.

**Patentansprüche**

1. Verfahren zum Spalten einer aliphatischen Etherverbindung durch Inberührungbringen mit einem Säurehalid in Gegenwart eines Lewis Säurekatalysators,
**dadurch gekennzeichnet,**
daß ein aliphatischer Ether, der mindestens ein Halogenatom in Beta-Stellung zum Sauerstoffatom aufweist, gespalten wird, und der entsprechende Ester und Halid mit dem (den) nicht umgesetzten Beta-Halogenatom(en) hergestellt werden.

2. Verfahren nach Anspruch 1,
**dadurch gekennzeichnet,**
daß der aliphatische Ether 3-12 Kohlenstoffatome aufweist.

3. Verfahren nach Anspruch 1,
**dadurch gekennzeichnet,**
daß ein 1,1'-Oxybis(2-halogen-$C_{2-6}$-Alkan) ge-

spalten wird.

4. Verfahren nach Anspruch 1,
**dadurch gekennzeichnet,**
daß ein 1,1'-Oxybis(1-halogenmethyl-2-halogen-$C_{2-5}$-Alkan) gespalten wird.

5. Verfahren nach Anspruch 1,
**dadurch gekennzeichnet,**
daß ein 1,1'-Oxybis(2-halogen-$C_{3-4}$-Alkan) oder 1,1'-Oxybis(1-halogenmethyl-2-halogen-$C_{2-3}$-Alkan) gespalten wird.

6. Verfahren nach Anspruch 1,
**dadurch gekennzeichnet,**
daß 1,1'-Oxybis(1-methyl-2-chlorethan) oder 1,1'-Oxybis(1-chlormethyl-2-chlorethan) gespalten wird.

7. Verfahren nach jedem der Ansprüche 1-6,
**dadurch gekennzeichnet,**
daß das Halogenatom in der Etherverbindung Chlor ist und das Säurehalid ein saures Chlorid oder saures Bromid ist.

8. Verfahren nach jedem der Ansprüche 1-7,
**dadurch gekennzeichnet,**
daß Acetylchlorid oder Acetylbromid als Säurehalid verwendet wird.

9. Verfahren zum Herstellen eines Diesters eines aliphatischen, benachbarten Glycols,
**dadurch gekennzeichnet,**
daß in einem ersten Schritt ein aliphatischer Ether mit mindestens einem Halogenatom in Beta-Stellung zum Sauerstoffatom mit einem Säurehalid gemäß dem Verfahren nach jedem der Ansprüche 1-8 gespalten wird und die Reaktionsprodukte in einem zweiten Schritt mit einem Alkalisalz oder einem Erdalkalisalz einer Carbonsäure oder Sulfonsäure zu dem (den) entsprechenden Ester(n) umgesetzt werden.

10. Verfahren nach Anspruch 9,
**dadurch gekennzeichnet,**
daß der aliphatische Ether ein Ether der Formel R-O-R ist, in der beide Reste R die gleiche Bedeutung haben und ein Halogenatom in Beta-Stellung zum Sauerstoffatom aufweisen, und weiterhin
**dadurch gekennzeichnet,**
daß das Säurehalid in dem ersten Schritt und das Alkalisalz oder Erdalkalisalz in dem zweiten Schritt von der gleichen Säure abgeleitet sind.